# EUROPEAN PATENT APPLICATION

(11) **EP 4 755 991 A1**
(43) Date of publication of application: **10.06.2026**
(21) Application number: 24851172.7
(22) Date of filing: 23.07.2024
(51) Int. Cl.: C09K 23/00, B01J 47/014, A23L 29/10, A61K 8/9794, A61K 36/899, A01N 25/30, C11D 1/00, C02F 1/26

(54) **PROTOCOL FOR OBTAINING A BIOSURFACTANT EXTRACT FROM CORN STEEP LIQUOR BY MEANS OF LIQUID-SOLID EXTRACTION, USING POLYMER SOLID MATRICES**

(30) Priority: 04.08.2023 ES 202300064
(71) Applicant: Universidade de Vigo, 36310 Vigo, Pontevedra (ES)
(72) Inventor: VECINO BELLO, Xanel, 36310 Vigo (PONTEVEDRA) (ES); CRUZ FREIRE, José Manuel, 36310 Vigo (PONTEVEDRA) (ES); MOLDES MENDUIÑA, Ana Belén, 36310 Vigo (PONTEVEDRA) (ES); MARTÍNEZ ARCOS, Andrea, 36310 Vigo (PONTEVEDRA) (ES)
(74) Representative: Clarke, Modet y Cía., S.L.
(86) International application number: PCT/ES2024/070472
(87) International publication number: WO 2025/032270

(57) **Abstract**

In this invention, the protocol to be followed for obtaining a biosurfactant extract from corn steep liquor is described, by separation using different solid polymeric matrices (liquid-solid extraction) and subsequent desorption of biosurfactant extract contained in the polymeric matrix by solid-liquid extraction The solvent used in the polymer regeneration and biosurfactant extract elution is separated by distillation or evaporation resulting in a solvent-free biosurfactant extract that is capable of reducing the surface tension of water by more than 15 units and with a critical micelle concentration (CMC), under the most favorable conditions, of less than 200 mg/L.

## Description

Biosurfactant separation from corn steep liquors by using solid polymer matrices that involve liquid-solid extraction processes and subsequent elution of biosurfactant extract with different eluents.

### TECHNICAL SECTOR

The present invention falls within the scope of obtaining naturally occurring surfactants from fermented liquid streams.

The main objective of this invention is to obtain a biosurfactant extract from corn steep liquors by liquid-solid extraction using different polymer matrices and subsequent desorption of biosurfactant extract using different eluents.

### BACKGROUND OF THE INVENTION

Corn steep liquors, produced as a by-product or secondary raw material during wet corn processing, are an interesting source of bioactive compounds including compounds having surfactant capacity which, being produced from a naturally and spontaneously fermented stream, have the connotation of biosurfactants. Biosurfactant compounds, unlike chemical surfactants, are made up of natural compounds produced by microorganisms or extracted from plant cells. In recent years, several methods have been collected for the extraction of these biosurfactants, obtaining different extracts depending on the proposed separation process. Extraction processes are covered in several patents (ES-2424399_B2, ES-2435324_B2, EP 28262624_B1-WO2014044876_A1, ES-2527366_B1, ES-2739050_B2, ES-2795574_A1).

The first publications related to the surfactant activity of corn steep liquors were disclosed in a patent (ES-2424399_B2) filed in 2012 and subsequently published in 2014 in the Journal Agricultural and Food Chemistry (Vecino et al. 2014). These publications do not refer to or propose methods of extracting biosurfactant extracts contained in corn steep liquors. "Only data demonstrating the surfactant capacity of corn washing liquors are provided, claiming in patent ES-2424399_B2 their direct use as a surface-active, detergent, or surfactant agent.

Subsequently, in national patent EP-28262624_B1 and European patent WO2014044876_A1 it is proposed to obtain biosurfactant extracts from corn steep liquors, using different extracting agents including the following solvents: isoamyl alcohol, chloroform, 1,2-dichloroethane, ethyl acetate, tetrahydrofuran, tributyl phosphate, methyl butyl ether, diethyl ether, trichloroethene, hexane, heptane, xylene, dichloromethane. **The** process comprises obtaining biosurfactant extracts by liquid-liquid extraction and subsequent separation of biosurfactant extract and solvent recovery by distillation. **The** uses of these biosurfactant extracts are included in several publications with potential application in the cosmetic industry, pharmaceutical industry and industry related to floor cleaning. **The** biosurfactant extracts obtained by this process consist of lipopeptides, phospholipids, as well as other biocompounds including antioxidants. The characterization of these extracts was published in 2020 in the journal Separation and Purification Technology (Rodriguez-Lopez et al. 2020); in addition, information related to the composition of these extracts is included in patent ES-2527366_B1. The extract obtained as reported in the literature has an oily brown-orange appearance (Knoth et al. 2019, International Journal of Pharmaceutics).

On the other hand, patent ES-2795574_A1 discloses the biosurfactant extraction from corn steep liquor using different buffered aqueous solutions, avoiding the use of organic solvents. In this patent, the biosurfactant extract extraction is carried out from the solid fraction contained in the corn steep liquors which includes microbial biomass, which contains bioactive compounds adhered to its plasma membrane that are extracted with the applied buffered solutions. After extraction of the biosurfactant compounds, the extract separated from the biomass is subjected to a dialysis process for the removal of salts and other low molecular weight compounds. After dialysis, the aqueous extract containing the biosurfactant compounds can be lyophilized for better preservation and management. The extract obtained, once lyophilized, is a white-yellow powder with a different composition, as disclosed in said patent, to that obtained following liquid-liquid extraction with organic solvents.

In addition, in corn steep liquors, the presence of phospholipids and lecithin has been detected, which are natural compounds with surfactant capacity, the extraction process of which is set out in patent ES-2739050_B2. Said patent claims the use of corn steep liquors as a source of lecithin and phospholipids for their application in the cosmetic and food field. The process applied in this invention for obtaining a biosurfactant extract based on lecithin and phospholipids consists of extraction with organic solvents (chloroform, ethyl acetate, etc.), followed by solvent distillation or evaporation and subsequent biosurfactant extract precipitation with acetone. In addition, and after the filing date of said patent, a study has been published in the journal Separation and Purification Technology (Rodriguez-Lopez et al. 2020) on the extraction and characterization of this biosurfactant extract based on phospholipids and lecithin extracted from corn steep liquor.

Regarding the use of liquid-solid extraction processes to obtain biosurfactants from corn steep liquors, a study was published in 2021 in the journal Water (Martinez-Arcos et al. 2021) that discloses the use of calcium alginate polymers and calcium alginate polymers combined with biologically oxidized grape bagasse for biosurfactant extraction from corn steep liquors, with extraction percentages of 55%.

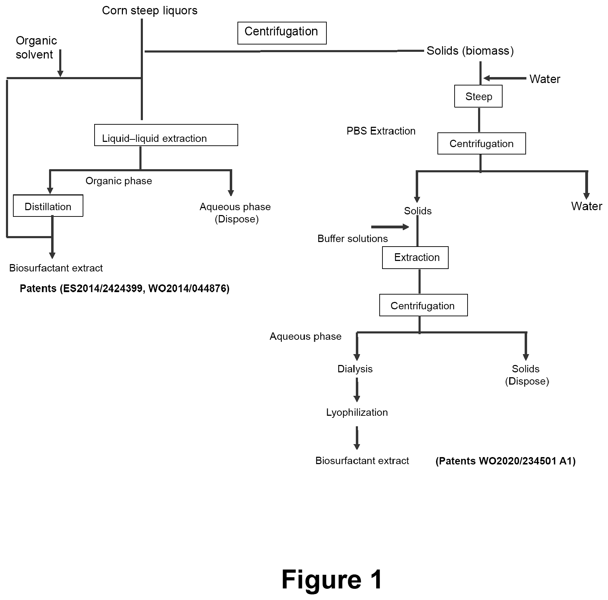

**Figure 1** shows in flowchart form the processes according to patents EP-28262624_B1-WO2014044876A1, ES-2795574_A1 for obtaining biosurfactants from corn steep liquors. This diagram shows 2 types of biosurfactant extracts. A first biosurfactant extract produced extracellularly and extracted from the liquid fraction of corn steep liquors by liquid-liquid extraction and a second biosurfactant extract obtained from the solid fraction of corn steep liquors by buffered aqueous solutions.

### EXPLANATION OF THE INVENTION

Different solid matrices of the commercial company Phenomenex are evaluated, for the biosurfactant extraction from corn steep liquors, selecting a weak anion exchange polymer that couples a hydrophobic backbone with a di-amino ligand containing primary and secondary amines (Strata-X-AW -Phenomenex), as the best option since it allows to fully recover the biosurfactants present in the corn steep liquors.

### DETAILED EXPLANATION OF THE INVENTION

First, a preselection of solid polymeric matrices of the commercial company Phenomenex (Strata-XL, Strata-XL-C, Strata-XL-CW, Strata-X-A, Strata-X-AW, Strata C18-E, Strata Phenyl, Strata NH₂) was carried out, whose characteristics are listed in **Table 1.**

To do this, the different solid polymeric matrices were contacted with the solid-free corn steep liquors at room temperature for 5 minutes, using different concentrations of solid matrix between 4 and 20 g/L.

Any solid-liquid separation process known in the art, such as centrifugation or filtration, can be used to separate the solid phase from the corn steep liquors.

**Table 1. Different solid polymeric matrices used with their characteristics.**

| **Polymeric solid matrix** | **Features** |
|---|---|
| Strata-XL | Reverse phase polymer matrix functionalized with hydrocarbon compounds. |
| Strata-XL-C | Strong cation exchange matrix, combining a hydrophobic backbone with a sulfonic acid functional group (-O₃S). |
| Strata-XL-CW | Weak cation exchange matrix, coupling a hydrophobic backbone with a carboxylic acid functional group (-O₂C). |
| Strata-X-A | Strong anion exchange polymer matrix, coupling a hydrophobic backbone with a dimethylbutylated quaternary amine functional group (⁺R₃N). |
| Strata-XL-A | Strong anion exchange matrix, coupling a hydrophobic backbone with a dimethylbutyl quaternary amine functional group (⁺R₃N). |
| Strata-X-AW | Weak anion exchange matrix, coupling a hydrophobic backbone with a diamino ligand containing primary and secondary amines (⁺H₃N). |
| Strata C18-E | Matrix offering strong hydrophobic retention with negligible secondary polar interactions with active silanol groups. |
| Strata Phenyl | Matrix with a short alkyl chain with a phenyl group providing moderate hydrophobic selectivity and aromatic selectivity through π-π interactions. |
| Strata NH₂ | Matrix offering strong polar selectivity and hydrogen bonding of hydrogen under normal phase conditions or can be used as anion exchange matrix (⁺H₃N). |

After contacting the corn steep liquor with the different solid polymeric matrices, the surface tension of liquors was measured in order to verify the presence or absence of biosurfactants.

Those matrices capable of extracting biosurfactants from corn steep liquors induced an increase in the surface tension of corn steep liquors upon contacting therewith. The surface tension of the solids-free corn steep liquors is around 50 mN/m, after contacting with the polymer solid matrices, this surface tension increased to 70-72 mN/m.

Among the evaluated solid polymeric matrices, the Strata-X-AW type solid matrices provided the best results, followed by Strata-X-A and Strata XL-C. The percentages of biosurfactant extraction detected in the corn steep liquors after contacting with the different solid polymeric matrices evaluated are shown in **Table 2.**

**Table 2. Percentage of entrapped biosurfactant by polymer solid matrices from corn steep liquors.**

| **Polymeric solid matrix** | **Percentage of extracted biosurfactant (%)** |
|---|---|
| Strata-XL | < 25 |
| Strata-XL-C | > 90 |
| Strata-XL-CW | < 50 |
| Strata-X-A | > 90 |
| Strata-X-AW | > 95 |
| Strata C18-E | < 50 |
| Strata Phenyl | < 50 |
| Strata NH₂ | > 85 |

As can be seen, the biosurfactant extract can be trapped by both anion and cation exchange matrices, which is consistent with its amphoteric character demonstrated in previous study (Rodriguez-Lopez et al. 2016) published in the Journal of Surfactants and Detergents. In this study, biosurfactant extracts were extracted from corn steep liquors with chloroform, and after distillation of the organic phase, the biosurfactant extract was diluted in water and contacted with Amberlite IRA 400 and Amberlite IR 120 resins, of anionic and cationic nature, respectively. After the liquid-solid extraction process, it was verified that both resins were able to extract the biosurfactant compounds present in the aqueous solution, reaching surface tension values of 72 mN/m. It should be noted that in this study the resins have not been applied on corn steep liquors but on the biosurfactant extract subjected to a chloroform extraction process in order to study its ionic character.

With the solid matrices that gave the best results, more extensive studies were carried out to optimize the operating conditions, testing different temperatures, including room temperature in the range of 18-27 °C, as well as 40 °C. Tests were also carried out with different solid-liquid ratios until the solid-liquid ratio was reduced to 0,01 g/mL (1:100 g/mL) and applying different extraction times (5 min, 30 min, 1 h, 4 h, 24 h).

Once the biosurfactants were trapped in the solid matrix, different eluents were tested in order to recover the biosurfactant extract.

The eluting agents tested were Milli-Q water, ethyl acetate, chloroform, ethanol, acetone, methanol, methanol + 5% formic acid, and methanol + 5% ammonia, which were kept in contact with the biosurfactant-loaded solid matrix for at least 5 minutes at temperatures between 18 and 40 °C, observing that an eluent:solid matrix ratio of 100:1 (mL/g) is sufficient to extract approximately 100% of the biosurfactant extract when the eluent is methanol in combination with ammonia or with formic acid. In addition, reuse tests of the solid matrix were carried out after 10 extraction and regeneration cycles, the losses in effectiveness of said solid matrix with respect to the liquid-solid extraction of biosurfactants being less than 25%.

On the other hand, different contact times between the eluent and biosurfactant-loaded solid matrix were tested between 5 and 60 min, observing that 5 min is sufficient time to extract the majority of the biosurfactants trapped by the polymer solid matrix.

The biosurfactant extract obtained has biomarkers with higher molecular weights than the biomarkers found in the biosurfactant extract protected under patents ES-2435324_B2, EP-28262624B1-WO2014044876A1, ES-2527366_B1, ES-2739050_B2, ES-2795574_A1, resulting in a more selective extract in terms of components.

### BRIEF DESCRIPTION OF THE DRAWINGS

In order to complement the description provided and with the aim of facilitating a better understanding of the features of the invention, a set of drawings is included as an integral part of said description, in which, by way of illustration and not limitation, the following is shown:
**Figure 1****.** Scheme of biosurfactant separation processes applied to corn steep liquors comprising a liquid-liquid extraction process with organic solvents or a solid-liquid extraction process with buffered aqueous solutions, applied in the latter case to the solid fraction of corn steep liquors. After the liquid-liquid extraction process, a distillation process necessary to recover the solvent and obtain the biosurfactant extract is included in this scheme. In relation to the solid-liquid extraction process applied to the solid fraction of corn steep liquors, a dialysis process that aims to remove salts present in the extracting agent and a freeze-drying process to obtain the biosurfactant extract in powder form are included.

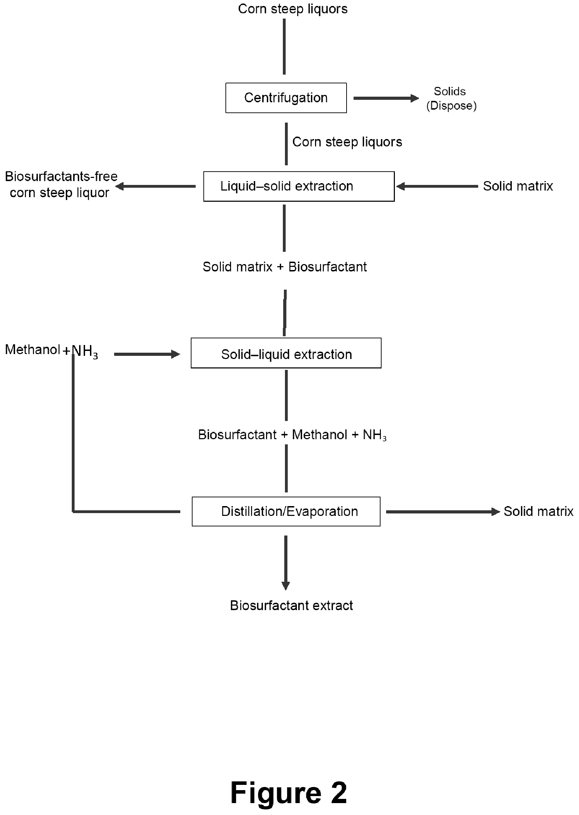

**Figure 2****.** Scheme of the novel extraction process proposed for biosurfactant separation from the liquid fraction of corn steep liquors using the Strata-AW type solid matrix, composed of a hydrophobic polymer bound to a di-amino ligand, which has been taken as an example. In this scheme, the process starts from corn steep liquor which, after centrifugation or filtration, is subjected to a liquid-solid extraction process with different polymeric matrices that retain the biosurfactant substances contained in the corn steep liquor. Subsequently, the polymeric matrices loaded with the surfactant substances are subjected to a solid-liquid extraction process using a solvent solution, for example based on methanol and NH₃. After the biosurfactant substance recovery, the solvent is subjected to a distillation or evaporation process in order to recover the solvent and separate it from the biosurfactant extract object of the invention.

### PREFERRED EMBODIMENT OF THE INVENTION

### Example 1. Obtaining the biosurfactant extract

Liquid-solid extractions were made to corn steep liquors supplied by FeedStimulants (Zoetermeer, The Netherlands), from the corn wet fractionation process. The corn steep liquors were taken and subjected to a centrifugation or filtration process, in order to obtain a solid-free liquid fraction, which was contacted with different solid polymeric matrices including the weak anionic polymer Strata-X-AW from Phenomenex, for a time of at least 5 minutes at room temperature.

**Figure 2** shows, by way of example, the scheme of the new proposed process for obtaining biosurfactants from corn steep liquors, by extraction with the Strata-AW type polymer solid matrix. This scheme includes the step of eluting the biosurfactant extract with a solution of methanol and NH₃, as well as recovering the solvent by evaporation or distillation.

## Claims

1. A process for biosurfactant extraction from corn steep liquors **characterized in that** it comprises the steps of separating the liquid fraction from corn steep liquors by separation processes with polymer solid matrices formed by a hydrophobic backbone and a di-amino ligand containing primary and secondary amines.

2. A process for biosurfactant extraction from corn steep liquors **characterized in that** it comprises the steps of separating the liquid fraction from corn steep liquors by separation processes with solid matrices formed by strong cation exchange matrices, combining a hydrophobic backbone with a sulfonic acid functional group.

3. A process for biosurfactant extraction from corn steep liquors **characterized in that** it comprises the steps of separating the liquid fraction from corn steep liquors by separation processes with polymer solid matrices formed by a strong anion exchange polymer matrix, coupling a hydrophobic backbone with a dimethylbutylated quaternary amine functional group.

4. The process for biosurfactant extraction from corn steep liquors according to claim 1, 2 or 3 **characterized in that** it further comprises eluting biosurfactants using the following solvents: ethyl acetate, chloroform, ethanol, acetone, methanol, formic acid, ammonia, in combination or separately.

5. The process for biosurfactant extraction from corn steep liquors according to claim 4 further comprising separating and recovering the solvent by distillation or evaporation.

6. Use or application of the biosurfactants obtained from the liquid fraction of the corn steep liquors, after subjecting them to the separation processes, according to claim 5 as a surfactant and/or emulsifying agent in the food, cosmetic, pharmaceutical, agrochemical or environmental industry.

7. Use or application of the biosurfactants obtained from the liquid fraction of the corn steep liquors, after subjecting them to the separation processes, according to claim 5 as surfactant and/or emulsifier agent in surface cleaning.

8. Use or application of the biosurfactants obtained from the liquid fraction of corn steep liquors, after subjecting them to the separation processes, according to claim 5 to improve the permeability of those formulas, containing active ingredients, through cell membranes, intended for cosmetic, pharmaceutical and agrochemical uses.
